# EUROPEAN PATENT APPLICATION

(11) **EP 4 710 847 A1**
(43) Date of publication of application: **18.03.2026**
(21) Application number: 24803495.1
(22) Date of filing: 08.05.2024
(51) Int. Cl.: A61B 5/022

(54) **BLOOD PRESSURE MEASUREMENT DEVICE AND BLOOD PRESSURE MEASUREMENT METHOD**

(30) Priority: 09.05.2023 JP 2023077429
(71) Applicant: Teikyo University, Tokyo 173-8605 (JP)
(72) Inventor: OGAWA, Mitsuhiro, Utsunomiya-shi, Tochigi 320-8551 (JP)
(74) Representative: Ström & Gulliksson AB
(86) International application number: PCT/JP2024/017096
(87) International publication number: WO 2024/232386

(57) **Abstract**

A blood pressure measurement device includes: a pressurizing unit configured to apply a pressure to a site of a subject to stop blood flow; a light detection unit configured to detect transmitted light that passes through a site distal to the site; and a control unit configured to determine an attenuation stop timing at which the transmitted light changes from an attenuation stage in which the transmitted light attenuates to an attenuation stop stage in which the attenuation of the transmitted light stops in a pressure increase stage in which the pressure increases, and output a blood pressure measurement value based on the pressure at the attenuation stop timing.

## Description

### TECHNICAL FIELD

The present disclosure relates to a blood pressure measurement device and a blood pressure measurement method.

Priority is claimed on Japanese Patent Application No. 2023-77429, filed May 9, 2023, the content of which is incorporated herein by reference.

### BACKGROUND ART

In the related art, a general blood pressure measurement device for home use captures arterial pulsation of a subject using an oscillometric method while changing a pressure (cuff pressure) inside a cuff wrapped around an upper arm or the like of a subject, and calculates a systolic blood pressure (so-called maximum blood pressure) and a diastolic blood pressure (so-called minimum blood pressure) (for example, see Patent Document 1).

### Citation List

### Patent Document

Patent Document 1: Japanese Unexamined Patent Application, First Publication No. H7-222722

### SUMMARY OF INVENTION

### Technical Problem

However, the above-described blood pressure measurement device in the related art exhibits low blood pressure measurement accuracy for a subject in whom pulsation is difficult to detect. For example, a patient implanted with a left ventricular assist device (LVAD) (patient with an LVAD) has a low arterial pressure in many cases, and it is difficult to measure a blood pressure with the above-described blood pressure measurement device in the related art.

The present disclosure has been made in consideration of such circumstances, and an object of the present disclosure is to improve the accuracy of blood pressure measurement for a subject in whom pulsation is difficult to detect.

### Solution to Problem

According to one aspect of the present disclosure, there is provided a blood pressure measurement device including: a pressurizing unit configured to apply a pressure to a site of a subject to stop blood flow; a light detection unit configured to detect transmitted light that passes through a site distal to the site; and a control unit configured to determine an attenuation stop timing at which the transmitted light changes from an attenuation stage in which the transmitted light attenuates to an attenuation stop stage in which the attenuation of the transmitted light stops in a pressure increase stage in which the pressure increases, and output a blood pressure measurement value based on the pressure at the attenuation stop timing.

According to one aspect of the present disclosure, in the above-described blood pressure measurement device, the control unit determines the attenuation stop timing by repeatedly increasing and decreasing the pressure when a slope of the attenuation of the transmitted light decreases to a value equal to or less than a predetermined slope.

According to one aspect of the present disclosure, in the above-described blood pressure measurement device, the control unit outputs a maximum blood pressure measurement value based on the pressure at the attenuation stop timing.

According to one aspect of the present disclosure, in the above-described blood pressure measurement device, the control unit determines an attenuation slope change timing at which a change in a slope of the attenuation of the transmitted light is equal to or greater than a predetermined change amount, and outputs a blood pressure measurement value based on the pressure at the attenuation slope change timing.

According to one aspect of the present disclosure, in the above-described blood pressure measurement device, the control unit outputs an average blood pressure measurement value based on the pressure at the attenuation slope change timing.

According to one aspect of the present disclosure, there is provided a blood pressure measurement device including: a pressurizing unit configured to apply a pressure to a site of a subject to stop blood flow; a light detection unit configured to detect transmitted light that passes through a site distal to the site; and a control unit configured to raise the pressure above a predetermined maximum blood pressure reference value and then decrease the pressure, determine the attenuation start timing at which the transmitted light starts to attenuate from a stage in which the transmitted light is constant, and output a blood pressure measurement value based on the pressure at the attenuation start timing.

According to one aspect of the present disclosure, the above-described blood pressure measurement device further includes: a light emitting unit configured to irradiate the distal site with light.

According to one aspect of the present disclosure, there is provided a blood pressure measurement method including: a pressurization step of, via a pressurizing unit, applying a pressure to a site of a subject to stop blood flow; a light detection step of, via a light detection unit, detecting transmitted light that passes through a site distal to the site; and a control step of, via a control unit, determining an attenuation stop timing at which the transmitted light changes from an attenuation stage in which the transmitted light attenuates to an attenuation stop stage in which the attenuation of the transmitted light stops in a pressure increase stage in which the pressure increases, and outputting a blood pressure measurement value based on the pressure at the attenuation stop timing.

According to one aspect of the present disclosure, there is provided a blood pressure measurement method including: a pressurization step of, via a pressurizing unit, applying a pressure to a site of a subject to stop blood flow; a light detection step of, via a light detection unit, detecting transmitted light that passes through a site distal to the site; and a control step of, via a control unit, raising the pressure above a predetermined maximum blood pressure reference value and then decreasing the pressure, determining an attenuation start timing at which the transmitted light starts to attenuate from a stage in which the transmitted light is constant, and outputting a blood pressure measurement value based on the pressure at the attenuation start timing.

### Advantageous Effects of Invention

According to the present disclosure, it is possible to obtain an effect of improving the accuracy of blood pressure measurement for a subject in whom pulsation is difficult to detect.

### BRIEF DESCRIPTION OF DRAWINGS

[FIG. 1] A block diagram showing an example configuration of a blood pressure measurement device according to an embodiment.
[FIG. 2] A diagram showing an example configuration of a light emitting unit and a light detection unit according to the embodiment.
[FIG. 3] A flowchart showing an example of a procedure of a blood pressure measurement method according to the embodiment.
[FIG. 4] A graph showing experimental results of the blood pressure measurement method according to the embodiment.

### DESCRIPTION OF EMBODIMENTS

Hereinafter, an embodiment of the present disclosure will be described with reference to the drawings. In the present embodiment, a hand will be described as an example of a blood pressure measurement site of a subject.

### [Overview]

When a cuff pressure of a cuff wrapped around, for example, an upper arm of the subject is in a range higher than a venous pressure and lower than a maximum blood pressure, arterial blood flows into and continues to pool in fingers of the subject, which are distal to the upper arm. Further, when the cuff pressure is increased and the cuff pressure is higher than the maximum blood pressure, blood does not flow into the arteries of the fingers, and thus no further pooling of blood occurs in the fingers. In the present embodiment, the blood pooling in the fingers is measured as a change in finger volume, and the maximum blood pressure is obtained from the measurement result. First, when a cuff pressure higher than the venous pressure is applied, the finger volume increases at first, but the finger volume stops changing at a time point at which the cuff pressure exceeds the maximum blood pressure. Therefore, in a case where the cuff pressure at a time point at which the finger volume stops changing is acquired, the acquired cuff pressure can be used as a measurement value of the maximum blood pressure. Here, for example, in a subject in whom pulsation is difficult to detect, such as a patient with an LVAD, the maximum blood pressure, the minimum blood pressure, and the average blood pressure are approximately equal. Therefore, by acquiring the cuff pressure at a time point at which the finger volume stops changing, it is possible to measure the maximum blood pressure and the minimum blood pressure in the same manner as in the general blood pressure measurement. The average blood pressure is an integrated average value of instantaneous values of the blood pressure.

As a method of measuring the volume of distal limbs such as fingers according to the present embodiment, transmission-type photoelectric plethysmography is used. In the present embodiment, a finger of the subject is irradiated with light, light that has passed through the finger (transmitted light) is detected, and a timing at which the finger volume stops changing is determined from a detection result of the transmitted light. The greater the amount of blood that pools in the finger, the more the transmitted light is attenuated. Therefore, as the finger volume increases, the amount of blood that pools in the finger increases, and thus the transmitted light is further attenuated. For this reason, a timing at which the attenuation of the transmitted light stops is used as the timing at which the finger volume stops changing. In the present embodiment, a change in volume at a site on a distal side away from a cuff, which is not in the vicinity of a site where a cuff pressure is applied by the cuff, is detected by the transmitted light. For example, a change in volume of a finger on the distal side away from the cuff, which is not in the vicinity of an upper arm where the cuff pressure is applied by the cuff, is detected by the transmitted light.

As described above, in the present embodiment, the finger of the subject is irradiated with light, light that has passed through the finger (transmitted light) is detected, the timing (attenuation stop timing) at which the attenuation of the transmitted light stops is determined from the detection result of the transmitted light, and the cuff pressure at the attenuation stop timing in the determination result is used as a blood pressure measurement value. As a result, the accuracy of blood pressure measurement for the subject in whom pulsation is difficult to detect is improved.

### [Blood Pressure Measurement Device]

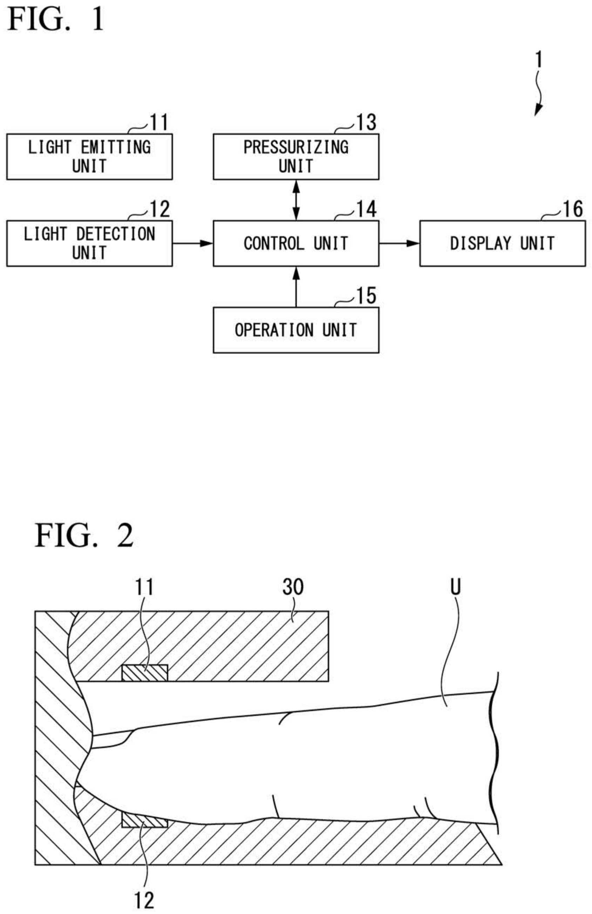

FIG. 1 is a block diagram showing an example configuration of a blood pressure measurement device according to an embodiment. In FIG. 1, a blood pressure measurement device 1 includes a light emitting unit 11, a light detection unit 12, a pressurizing unit 13, a control unit 14, an operation unit 15, and a display unit 16.

The pressurizing unit 13 applies a pressure to a site (pressurized site) of the subject to stop blood flow. For example, the pressurizing unit 13 applies a pressure to stop blood flow in the upper arm of the subject by increasing a pressure (cuff pressure) inside a cuff wrapped around the upper arm of the subject. The site to which the cuff pressure is applied is not limited to the upper arm. For example, the site to which the cuff pressure is applied may be a forearm or wrist.

The light emitting unit 11 emits light to a site (distal site) distal to the pressurized site of the subject. The light detection unit 12 detects light (transmitted light) that passes through the distal site.

FIG. 2 is a diagram showing an example configuration of a light emitting unit and a light detection unit according to the embodiment. In the example of FIG. 2, the distal site is a fingertip of a subject U. The light emitting unit 11 is, for example, a light emitting element such as a light emitting diode (LED). The light detection unit 12 is, for example, a light receiving element such as CdS, a photodiode, or a phototransistor. In FIG. 2, the light emitting unit 11 and the light detection unit 12 are installed in a measurement jig 30. In a case where the subject U places the fingertip at a predetermined position of the measurement jig 30, the light emitting unit 11 and the light detection unit 12 are installed in the measurement jig 30 such that the light from the light emitting unit 11 is incident on the fingertip and transmitted light that has passed through the fingertip is detected by the light detection unit 12.

The light emitted from the light emitting unit 11 has a wavelength that is attenuated by blood. Specifically, the light emitted from the light emitting unit 11 has a wavelength that is attenuated by hemoglobin in blood. The light emitted from the light emitting unit 11 is, for example, red or near infrared light. The wavelength of the light emitted by the light emitting unit 11 is, for example, 808 nanometers.

The light detection unit 12 outputs a received light intensity signal indicating the intensity of the detected transmitted light to the control unit 14.

Hereinafter, an example configuration of FIG. 2 will be described.

The control unit 14 controls blood pressure measurement. The control unit 14 determines an attenuation stop timing at which the transmitted light that passes through the fingertip of the subject U changes from an attenuation stage in which the transmitted light attenuates to an attenuation stop stage in which the attenuation of the transmitted light stops in a pressure increase stage in which the cuff pressure applied to the pressurized site of the subject U is increased by the pressurizing unit 13, and outputs a blood pressure measurement value based on the cuff pressure at the attenuation stop timing.

The control unit 14 includes computer hardware such as a CPU and a memory, and functions of the control unit 14 are realized by the CPU executing a computer program stored in the memory.

The operation unit 15 includes an input device such as an operation key or a numeric keypad that is operated by a user (for example, a subject or a family of the subject) of the blood pressure measurement device 1, and inputs data according to the operation of the user. The operation unit 15 outputs the data input by the input device to the control unit 14. The display unit 16 is configured of, for example, a display device such as a liquid crystal display device, and displays data such as a blood pressure measurement menu and a blood pressure measurement value output from the control unit 14. The blood pressure measurement device 1 may include a touch panel that allows for both data input and data display.

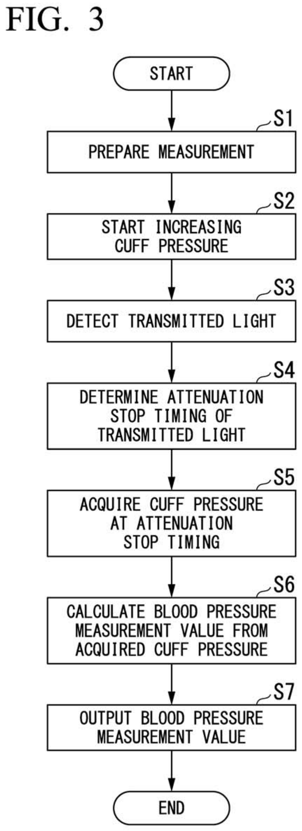

Next, a blood pressure measurement method according to the present embodiment will be described with reference to FIG. 3. FIG. 3 is a flowchart showing an example of a procedure of the blood pressure measurement method according to the present embodiment. Here, a case will be described as an example in which the subject U himself/herself is a user of the blood pressure measurement device 1 and operates the blood pressure measurement device 1. The user of the blood pressure measurement device 1 (a person who operates the blood pressure measurement device 1) and the subject U may be different persons.

(Step S1) The subject U prepares for blood pressure measurement. For example, the blood pressure measurement device 1 is powered on, a cuff is wrapped around a pressurized site (for example, an upper arm), or a finger is placed on a predetermined position of the measurement jig 30. The control unit 14 of the blood pressure measurement device 1 performs initialization of the device when the blood pressure measurement device 1 is powered on. This initialization enables the blood pressure measurement in the blood pressure measurement device 1. The control unit 14 causes the display unit 16 to display a blood pressure measurement menu. The light emitting unit 11 irradiates the fingertip of the subject U with light.

(Step S2) The subject U uses the operation unit 15 to select blood pressure measurement start from the blood pressure measurement menu. In a case where the blood pressure measurement start is selected, the control unit 14 instructs the pressurizing unit 13 to start increasing the cuff pressure. The pressurizing unit 13 increases the cuff pressure in response to an instruction from the control unit 14 to start increasing the cuff pressure. The pressurizing unit 13 continues to increase the cuff pressure until the pressurizing unit 13 receives an instruction from the control unit 14 to stop increasing the cuff pressure. The pressurizing unit 13 always outputs a cuff pressure signal indicating the cuff pressure to the control unit 14. The control unit 14 records the cuff pressure signal in association with the measurement time point.

(Step S3) The light detection unit 12 detects transmitted light that has passed through the fingertip of the subject U, and outputs a received light intensity signal indicating the intensity of the detected transmitted light to the control unit 14.

(Step S4) The control unit 14 determines the attenuation stop timing of the transmitted light based on the received light intensity signal output from the light detection unit 12. For example, the control unit 14 calculates a slope of attenuation of the intensity of the transmitted light indicated by the received light intensity signal, and determines the attenuation stop timing of the transmitted light when the slope decreases to a value equal to or less than a predetermined slope. For example, a time point at which the slope decreases to the value equal to or less than the predetermined slope may be determined as the attenuation stop timing of the transmitted light. For example, a time point at which the slope has continuously remained at the value equal to or less than the predetermined slope for a predetermined period of time may be determined as the attenuation stop timing of the transmitted light.

In addition, when the slope of the attenuation of the transmitted light decreases to the value equal to or less than the predetermined slope, the control unit 14 may determine the attenuation stop timing by causing the pressurizing unit 13 to repeatedly increase and decrease the cuff pressure.

The control unit 14 instructs the pressurizing unit 13 to stop increasing the cuff pressure after determining the attenuation stop timing of the transmitted light. The pressurizing unit 13 stops increasing the cuff pressure in response to an instruction from the control unit 14 to stop increasing the cuff pressure.

(Step S5) The control unit 14 acquires the cuff pressure at the measurement time point corresponding to the attenuation stop timing. Specifically, the control unit 14 acquires, from among the cuff pressure signals recorded by itself, a cuff pressure signal that is recorded in association with the measurement time point corresponding to the attenuation stop timing. The control unit 14 may acquire cuff pressure signals at a plurality of measurement time points that include the measurement time point corresponding to the attenuation stop timing as well as time points before and after that time point.

(Step S6) The control unit 14 calculates the blood pressure measurement value from the cuff pressure indicated by the acquired cuff pressure signal. For example, the control unit 14 may set the cuff pressure indicated by the cuff pressure signal at the measurement time point corresponding to the attenuation stop timing as a maximum blood pressure measurement value. For example, the control unit 14 may perform statistical processing (for example, averaging) on a plurality of cuff pressures indicated by the cuff pressure signals at a plurality of measurement time points that include the measurement time point corresponding to the attenuation stop timing as well as time points before and after that time point, and set the cuff pressure obtained as a result of the statistical processing as the maximum blood pressure measurement value. For example, when a measurement mode for a subject in whom pulsation is difficult to detect, such as a patient with an LVAD, is selected from the blood pressure measurement menu, the control unit **14** may also use the maximum blood pressure measurement value as a measurement value of the minimum blood pressure or the average blood pressure (the maximum blood pressure measurement value = the minimum blood pressure measurement value = the average blood pressure measurement value).

(Step S7) The control unit **14** outputs the blood pressure measurement value. The display unit 16 displays the blood pressure measurement value output from the control unit 14. For example, the display unit 16 displays the maximum blood pressure measurement value output from the control unit 14. For example, the display unit 16 displays the maximum blood pressure measurement value and the minimum blood pressure measurement value output from the control unit 14. For example, the display unit 16 displays the maximum blood pressure measurement value, the minimum blood pressure measurement value, and the average blood pressure measurement value output from the control unit 14.

The control unit **14** outputs the blood pressure measurement value and also instructs the pressurizing unit 13 to release the cuff pressure. The pressurizing unit 13 releases the cuff pressure in response to an instruction from the control unit **14** to release the cuff pressure. As a result, the subject U recognizes his or her own blood pressure measurement value, and removes the finger from the predetermined position of the measurement jig 30 or removes the cuff, thereby completing the blood pressure measurement.

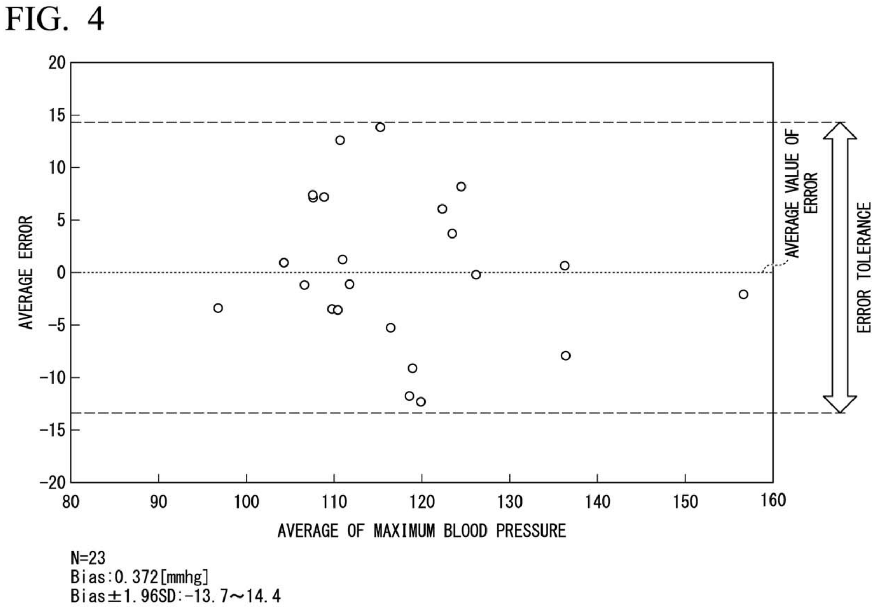

FIG. 4 is a graph showing experimental results of the blood pressure measurement method according to the present embodiment. FIG. 4 shows a graph of a "Bland-Altman plot" created using a measurement value of the maximum blood pressure using the blood pressure measurement method according to the present embodiment and a measurement value of the maximum blood pressure using the oscillometric method in the related art. The graph of the "Bland-Altman plot" of FIG. 4 shows that the blood pressure measurement method according to the present embodiment is equivalent to the oscillometric method in the related art.

### (Another Example 1 of Blood Pressure Measurement Method)

Here, the blood pressure measurement is performed based on a change in the slope of the attenuation of the transmitted light. In a case of the average blood pressure, there is a strong relationship between a change in finger volume and a change in cuff pressure, and a constant volume change can be obtained with a small change in cuff pressure. Therefore, in the average blood pressure, the change in finger volume suddenly increases due to a slight increase or decrease in cuff pressure, so that the slope of the attenuation of the transmitted light suddenly changes. Therefore, it is possible to monitor the change in the slope of the attenuation of the transmitted light, determine a timing (attenuation slope change timing) at which the slope of the attenuation of the transmitted light suddenly increases, and use the cuff pressure at the attenuation slope change timing as a measurement value of the average blood pressure.

Specifically, the control unit 14 determines the attenuation slope change timing of the transmitted light based on the received light intensity signal output from the light detection unit 12. For example, the control unit 14 calculates a slope of attenuation of the intensity of the transmitted light indicated by the received light intensity signal, and determines the attenuation slope change timing of the transmitted light when a change in the slope is equal to or greater than a predetermined change amount. For example, a time point at which the change in the slope is equal to or greater than the predetermined change amount may be determined as the attenuation slope change timing of the transmitted light. For example, a time point at which the change in the slope has continuously remained at the value equal to or greater than the predetermined change amount for a predetermined period of time may be determined as the attenuation slope change timing of the transmitted light.

The control unit 14 instructs the pressurizing unit 13 to stop increasing the cuff pressure after determining the attenuation slope change timing of the transmitted light. The pressurizing unit 13 stops increasing the cuff pressure in response to an instruction from the control unit 14 to stop increasing the cuff pressure.

The control unit 14 acquires the cuff pressure at the measurement time point corresponding to the attenuation slope change timing. Specifically, the control unit 14 acquires, from among the cuff pressure signals recorded by itself, a cuff pressure signal that is recorded in association with the measurement time point corresponding to the attenuation slope change timing. The control unit 14 may acquire cuff pressure signals at a plurality of measurement time points that include the measurement time point corresponding to the attenuation slope change timing as well as time points before and after that time point.

The control unit 14 calculates the blood pressure measurement value from the cuff pressure indicated by the acquired cuff pressure signal. For example, the control unit 14 may set the cuff pressure indicated by the cuff pressure signal at the measurement time point corresponding to the attenuation stop timing as an average blood pressure measurement value.

### (Another Example 2 of Blood Pressure Measurement Method)

In the above-described blood pressure measurement method, the cuff pressure is increased from a cuff pressure lower than the maximum blood pressure, the attenuation stop timing at which the attenuation of the transmitted light stops is determined, and the cuff pressure at the attenuation stop timing of the determination result is used as the blood pressure measurement value (for example, the maximum blood pressure measurement value), but the reverse procedure may also be used. Specifically, the cuff pressure is once raised above a predetermined maximum blood pressure reference value and then decreased, a timing (attenuation start timing) in which the transmitted light starts to attenuate from a stage in which the transmitted light is constant is determined, and the cuff pressure at the attenuation start timing in the determination result is used as the blood pressure measurement value (for example, the maximum blood pressure measurement value).

According to the above-described embodiment, it is possible to improve the accuracy of blood pressure measurement for a subject in whom pulsation is difficult to detect, such as a patient with an LVAD. In addition, the blood pressure measurement device 1 according to the present embodiment is also effective for a subject having severely low blood pressure, or a subject for whom it is difficult to measure blood pressure with a home blood pressure measurement device using the oscillometric method in the related art.

In the above-described embodiment, the blood pressure measurement device 1 includes the light emitting unit 11; however, when blood pressure measurement is performed in a place where a certain amount of light can be obtained, the light emitting unit 11 does not need to be provided.

The embodiment of the present disclosure has been described above in detail with reference to the drawings. However, the specific configurations are not limited to the embodiment, and include a design modification or the like within a scope which does not depart from the gist of the present disclosure.

In addition, a computer program for realizing the functions of the blood pressure measurement device 1 described above may be recorded on a computer-readable recording medium, and the program recorded on the recording medium may be read by the computer system and executed. Note that "computer system" mentioned herein may include hardware such as OS and peripheral devices.

In addition, the term "computer-readable recording medium" refers to a storage device, such as a writable non-volatile memory such as a flexible disk, a magneto-optical disk, an ROM, or a flash memory, a portable medium such as a digital versatile disc (DVD), or a built-in hard disk in a computer system.

Furthermore, the term "computer-readable recording medium" also includes a recording medium that holds a program for a certain period of time, such as a volatile memory (for example, dynamic random access memory (DRAM)) in a computer system that serves as a server or client in a case where a program is transmitted via a network such as the Internet or a communication channel such as a telephone circuit.

In addition, the program may be transmitted from the computer system in which the program is stored in the storage device or the like to another computer system via a transmission medium or by a transmission wave in the transmission medium. Here, the term "transmission medium" that transmits the program refers to a medium having a function of transmitting information such as a network (communication network) such as the Internet or a communication channel (communication line) such as a telephone line.

In addition, the program may be a program for realizing some of the functions described above. Furthermore, the program may be a so-called difference file (difference program) that can realize the above-described functions in combination with a program already recorded in the computer system.

### REFERENCE SIGNS LIST

1 Blood pressure measurement device
11 Light emitting unit
12 Light detection unit
13 Pressurizing unit
14 Control unit
15 Operation unit
16 Display unit

## Claims

1. A blood pressure measurement device comprising:
a pressurizing unit configured to apply a pressure to a site of a subject to stop blood flow;
a light detection unit configured to detect transmitted light that passes through a site distal to the site; and
a control unit configured to determine an attenuation stop timing at which the transmitted light changes from an attenuation stage in which the transmitted light attenuates to an attenuation stop stage in which the attenuation of the transmitted light stops in a pressure increase stage in which the pressure increases, and output a blood pressure measurement value based on the pressure at the attenuation stop timing.

2. The blood pressure measurement device according to Claim 1,
wherein the control unit determines the attenuation stop timing by repeatedly increasing and decreasing the pressure when a slope of the attenuation of the transmitted light decreases to a value equal to or less than a predetermined slope.

3. The blood pressure measurement device according to Claim 1 or 2,
wherein the control unit outputs a maximum blood pressure measurement value based on the pressure at the attenuation stop timing.

4. The blood pressure measurement device according to Claim 1,
wherein the control unit determines an attenuation slope change timing at which a change in a slope of the attenuation of the transmitted light is equal to or greater than a predetermined change amount, and outputs a blood pressure measurement value based on the pressure at the attenuation slope change timing.

5. The blood pressure measurement device according to Claim 4,
wherein the control unit outputs an average blood pressure measurement value based on the pressure at the attenuation slope change timing.

6. A blood pressure measurement device comprising:
a pressurizing unit configured to apply a pressure to a site of a subject to stop blood flow;
a light detection unit configured to detect transmitted light that passes through a site distal to the site; and
a control unit configured to raise the pressure above a predetermined maximum blood pressure reference value and then decrease the pressure, determine an attenuation start timing at which the transmitted light starts to attenuate from a stage in which the transmitted light is constant, and output a blood pressure measurement value based on the pressure at the attenuation start timing.

7. The blood pressure measurement device according to Claim 1 or 6, further comprising:
a light emitting unit configured to irradiate the distal site with light.

8. A blood pressure measurement method comprising:
a pressurization step of, via a pressurizing unit, applying a pressure to a site of a subject to stop blood flow;
a light detection step of, via a light detection unit, detecting transmitted light that passes through a site distal to the site; and
a control step of, via a control unit, determining an attenuation stop timing at which the transmitted light changes from an attenuation stage in which the transmitted light attenuates to an attenuation stop stage in which the attenuation of the transmitted light stops in a pressure increase stage in which the pressure increases, and outputting a blood pressure measurement value based on the pressure at the attenuation stop timing.

9. A blood pressure measurement method comprising:
a pressurization step of, via a pressurizing unit, applying a pressure to a site of a subject to stop blood flow;
a light detection step of, via a light detection unit, detecting transmitted light that passes through a site distal to the site; and
a control step of, via a control unit, raising the pressure above a predetermined maximum blood pressure reference value and then decreasing the pressure, determining an attenuation start timing at which the transmitted light starts to attenuate from a stage in which the transmitted light is constant, and outputting a blood pressure measurement value based on the pressure at the attenuation start timing.
